# EUROPEAN PATENT APPLICATION

(11) **EP 4 825 866 A1**
(43) Date of publication of application: **21.10.2026**
(21) Application number: 25895370.2
(22) Date of filing: 31.10.2025
(51) Int. Cl.: C07K 7/06, A61K 38/08, A61P 17/00, A61K 8/64, A61Q 19/00

(54) **MULTIFUNCTIONAL PEPTIDE, AND COMPOSITION COMPRISING SAME FOR IMPROVING SKIN CONDITION**

(30) Priority: 20.11.2024 KR 20240165774; 30.10.2025 KR 20250160659
(71) Applicant: Skinmed Co., Ltd., Daejeon 34050 (KR)
(72) Inventor: BANG, Jin Ho, Cheongju-si, Chungcheongbuk-do 28443 (KR); KIM, Miyoon, Daejeon 35314 (KR); YUN, Jae Nam, Daejeon 34066 (KR); HWANG, Yullye, Daejeon 34955 (KR); LEE, Jeung Hoon, Daejeon 34124 (KR)
(74) Representative: Kraus & Lederer PartGmbB
(86) International application number: PCT/KR2025/017726
(87) International publication number: WO 2026/111243

(57) **Abstract**

The novel hexapeptide having the amino acid sequence represented by SEQ ID NO: 1 of the present invention is a multi-functional peptide that simultaneously realizes inhibition of extracellular matrix (Extracellular Matrix, ECM) degradation, promotion of ECM synthesis, and inhibition of an inflammatory response compared with conventional commercial peptides (Matrixyl^{™}, SYN^{™}-AKE, etc.) despite being a single ingredient, and can provide an integrated effect capable of simultaneously improving various causes of skin aging.

## Description

### Technical Field

The present invention relates to a multi-functional peptide and a composition for improving a skin condition comprising the same, and more specifically, the peptide (SEQ ID NO: 1) exhibits an effect of integrally improving complex skin aging problems by inhibiting degradation of the skin extracellular matrix (Extracellular Matrix, ECM), promoting synthesis thereof, and simultaneously regulating an inflammatory response.

### Background Art

Skin aging is a biological phenomenon that occurs through the combined action of intrinsic factors associated with increasing age and photoaging caused by external environmental factors such as ultraviolet exposure. A core characteristic of such aging process is gradual destruction and functional deterioration of the extracellular matrix (Extracellular Matrix, ECM), which maintains the structure and elasticity of the skin. Collagen and elastin, which are major components of the ECM, are degraded by matrix metalloproteinases (Matrix Metalloproteinases, MMPs) such as collagenase and elastase, respectively, and at the same time, synthesis thereof is reduced due to deterioration in the function of fibroblasts.

Various technologies have been developed to respond to such complex skin aging mechanisms, but they have clear limitations. For example, retinoids, which are derivatives of vitamin A, are known to improve wrinkles by inhibiting MMP production, but may cause skin irritation and have limitations in directly promoting collagen biosynthesis.

In addition, Matrixyl^{™} of Sederma (INCI: palmitoyl pentapeptide-4, U.S. Patent No. 6,620,419), which is known in the field of functional peptides, and heptapeptides disclosed in Korean Patent Publication No. 10-2018-0108984 show an effect of promoting collagen synthesis, but their effect of directly inhibiting MMP activity is insignificant, and thus a complete wrinkle-improving effect is difficult to expect. Furthermore, ingredients that alleviate expression wrinkles through neurotransmitter regulation, such as Argireline^{™} (INCI: acetyl hexapeptide-8) and SYN^{™}-AKE (INCI: dipeptide diaminobutyroyl benzylamide diacetate), do not provide a direct solution to fundamental collapse of the ECM or the phenomenon of inflammaging.

Accordingly, the prior art has merely fragmentarily solved only some of the three core tasks of inhibiting ECM degradation, promoting ECM synthesis, and controlling inflammaging. Thus, there is a need to develop a multi-functional material capable of comprehensively improving skin elasticity and wrinkles by simultaneously controlling the three pathways.

### Disclosure of Invention

### Technical Problem

An object of the present invention is to overcome limitations of the prior art. Existing active ingredients for preventing and improving skin aging have been mainly limited to fragmentary functions of promoting synthesis of the extracellular matrix (ECM) or inhibiting degradation thereof, and have had a limitation in that they cannot effectively control inflammatory responses that accelerate aging.

Accordingly, an object of the present invention is to provide a novel multi-functional peptide, as a single ingredient, capable of simultaneously inhibiting and improving three core pathways that are complex causes of skin aging, namely (1) degradation of extracellular matrix (ECM) such as collagen and elastin, (2) reduction in collagen synthesis, and (3) chronic inflammatory response, and a composition comprising the same.

In addition, another object of the present invention is to provide a composition capable of contributing not only to improvement of skin aging, but also to prevention or improvement of various inflammatory skin diseases or skin conditions such as acne, atopic dermatitis, and sensitive skin, by utilizing the anti-inflammatory effect of the peptide.

### Solution to Problem

In order to solve the above problems, there is provided a hexapeptide having the RKWRYR sequence represented by SEQ ID NO: 1. (Provided that R is arginine, K is lysine, W is tryptophan, and Y is tyrosine.)

As used herein, the term "peptide" means a linear molecule formed by amino acid residues linked by peptide bonds. The peptide may be prepared by various methods including conventional biological or chemical synthesis methods known in the art, particularly solid-phase peptide synthesis (SPPS).

The present invention may comprise an amino acid sequence substantially identical to the amino acid sequence of SEQ ID NO: 1. Variants or fragments of amino acids having different sequences due to deletion, insertion, substitution, or a combination thereof of amino acid residues within a range that does not affect the function of the peptide are also included within the scope of the present invention. Variations in which the activity of the peptide is maintained as a whole by amino acid exchange are widely known in the art.

In addition, in some cases, the peptide may include chemical modifications such as phosphorylation, sulfation, acrylation, glycosylation, methylation, farnesylation, and palmitoylation. Accordingly, the present invention includes a peptide comprising the amino acid sequence of SEQ ID NO: 1, a variant having an amino acid sequence substantially identical thereto, or an active fragment thereof.

As used herein, "substantially identical amino acid sequence" means a sequence having sequence homology of 60% or more, preferably 75% or more, more preferably 80% or more, still more preferably 90% or more, most preferably 95% or more, for example 98% or more, or 99% or more with the amino acid sequence of SEQ ID NO: 1, while exhibiting the same biological activity. Furthermore, the peptide of SEQ ID NO: 1 of the present invention may additionally comprise a targeting sequence, a tag, a labeling residue, a half-life increasing sequence, a stability-enhancing sequence, and the like.

In addition, the peptide may be prepared by various methods widely known in the art. For example, in one embodiment of the present invention, the peptide may be prepared using polynucleotide recombination and a protein expression system, or may be prepared through chemical synthesis in vitro (for example, solid-phase synthesis) and cell-free protein synthesis.

In addition, the present invention includes a derivative of a hexapeptide having the amino acid sequence represented by SEQ ID NO: 1.

In the present invention, "derivative of a peptide" means a substance that substantially maintains the original activity, that is, inhibitory activity against extracellular matrix (ECM) degradation, collagen synthesis-promoting activity, and anti-inflammatory activity, even if a part of the amino acid sequence of the peptide is substituted, added, or deleted, or even if the N-terminus or C-terminus is modified. For example, those in which an acetyl group, a palmitoyl group, or the like is added to a terminus of the peptide in order to improve stability or skin permeability, or those modified in an amide form, may be included therein. In particular, the peptide of SEQ ID NO: 1 of the present invention is preferably in the form of a palmitoyl-peptide in which a palmitoyl group is added to the N-terminus in order to improve stability and skin permeability. This is intended to maximize, within the skin, the effect of the unique amino acid sequence possessed by the peptide of SEQ ID NO: 1 of the present invention.

The hexapeptide of the present invention may be a hexapeptide in which the N-terminus is modified with a palmitoyl group.

The present invention provides a pharmaceutical composition for preventing or treating an inflammatory skin disease, comprising a hexapeptide having the amino acid sequence represented by SEQ ID NO: 1 or a hexapeptide having the amino acid sequence represented by SEQ ID NO: 1 in which the N-terminus is modified with a palmitoyl group.

The hexapeptide may exhibit an anti-inflammatory action by inhibiting production of nitric oxide (Nitric Oxide, NO) or prostaglandin E2 (Prostaglandin E2, PGE2). In addition, the hexapeptide may strengthen the skin barrier by inhibiting extracellular matrix (Extracellular Matrix, ECM) degradation through inhibition of the activity of collagenase or elastase, or by promoting ECM synthesis through promotion of collagen synthesis in fibroblasts.

The inflammatory skin disease may be at least one selected from acne, atopic dermatitis, contact dermatitis, allergic dermatitis, seborrheic dermatitis, psoriasis, photosensitive dermatitis, and rosacea.

In the pharmaceutical composition for preventing or treating an inflammatory skin disease of the present invention, the acne, atopic dermatitis, contact dermatitis, allergic dermatitis, seborrheic dermatitis, psoriasis, photosensitive dermatitis, and rosacea are skin diseases that progress due to inflammation, and these inflammatory skin diseases may be treated or improved by inhibiting production of nitric oxide (Nitric Oxide, NO) or prostaglandin E2 (Prostaglandin E2, PGE2) to reduce inflammatory responses, by inhibiting degradation of the extracellular matrix, or by promoting ECM synthesis.

The peptide of SEQ ID NO: 1 may be added in an amount of preferably 0.001 to 50% by weight, more preferably 0.001 to 40% by weight, and most preferably 0.001 to 30% by weight, based on the total weight of the entire pharmaceutical composition.

The pharmaceutical composition may be formulated and used, according to conventional methods, in the form of formulations such as suspensions, emulsions, solutions, and aerosols, external preparations, suppositories, and sterile injectable solutions. Carriers, excipients, and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. In formulation, the composition is prepared using ordinarily used fillers, extenders, binders, wetting agents, disintegrants, surfactants, sweeteners, acidulants, and other diluents or excipients. In addition, wetting agents, sweeteners, flavoring agents, preservatives, acidulants, and the like may be included. Further, non-aqueous solvents and suspending agents may include propylene glycol, polyethylene glycol, vegetable oils such as olive oil, and injectable esters such as ethyl oleate.

The dose of the pharmaceutical composition of the present invention will vary depending on the age, sex, and body weight of the subject to be treated, the specific disease or pathological condition to be treated, the severity of the disease or pathological condition, the route of administration, and the judgment of the prescriber. Determination of the dose based on these factors is within the level of those skilled in the art, and generally the dose is in the range of 0.01 mg/kg/day to approximately 500 mg/kg/day. A preferred dose is 0.1 mg/kg/day to 200 mg/kg/day, and a more preferred dose is 1 mg/kg/day to 200 mg/kg/day. Administration may be carried out once a day or divided into several administrations. The above dose does not limit the scope of the present invention in any respect.

The present invention provides a cosmetic composition for improving a skin condition, comprising a hexapeptide having the amino acid sequence represented by SEQ ID NO: 1 or a hexapeptide having the amino acid sequence represented by SEQ ID NO: 1 in which the N-terminus is modified with a palmitoyl group. The improvement of the skin condition is characterized by improvement of skin elasticity, improvement of skin wrinkles, prevention of skin aging, enhancement of skin elasticity, or improvement of inflammatory skin. In addition, the improvement of inflammatory skin may be improvement of acne, atopic dermatitis, contact dermatitis, allergic dermatitis, seborrheic dermatitis, psoriasis, photosensitive dermatitis, or rosacea.

The improvement of the skin condition may be caused by at least one mechanism selected from the group consisting of inhibition of extracellular matrix (Extracellular Matrix, ECM) degradation, promotion of ECM synthesis, and inhibition of an inflammatory response. The inhibition of extracellular matrix (Extracellular Matrix, ECM) degradation may be inhibition of collagenase activity or inhibition of elastase activity. The promotion of ECM synthesis may be promotion of collagen synthesis in fibroblasts. The inhibition of an inflammatory response may be inhibition of production of nitric oxide (Nitric Oxide, NO) or inhibition of production of prostaglandin E2 (Prostaglandin E2, PGE2).

The cosmetic composition of the present invention may comprise, in addition to the peptide of SEQ ID NO: 1, ingredients conventionally used in cosmetic compositions, and may comprise conventional adjuvants and carriers such as antioxidants, stabilizers, solubilizers, vitamins, pigments, and fragrances.

The cosmetic composition of the present invention may be prepared in any formulation conventionally prepared in the industry, and may be formulated, for example, as a solution, suspension, emulsion, paste, gel, cream, lotion, powder, powder foundation, emulsion foundation, wax foundation, spray, and the like, but is not limited thereto. More specifically, the cosmetic composition of the present invention may be prepared in the form of skin lotion, essence, ampoule, serum, lotion, cream, pack, gel, spray, or powder. Compositions of these formulations may be prepared according to conventional methods in the art.

The cosmetic composition of the present invention may also be applied to highly functional formulations such as microneedle patches, hydrogel masks, and scalp tonics, which enhance skin delivery efficiency of the active ingredient, and may be applied to fillers and the like.

In addition, the cosmetic composition according to the present invention may comprise other ingredients, in addition to the peptide of SEQ ID NO: 1, within a range that does not impair the main effect, and preferably ingredients capable of providing a synergistic effect on the main effect. In addition, the composition according to the present invention may further comprise a humectant, emollient, ultraviolet absorber, preservative, bactericide, antioxidant, pH adjuster, organic and inorganic pigments, fragrance, cooling agent, or limiting agent. The blending amount of the above ingredients may be easily selected by those skilled in the art within a range that does not impair the purpose and effect of the present invention.

The novel hexapeptide having the amino acid sequence represented by SEQ ID NO: 1 of the present invention is a multi-functional peptide that simultaneously realizes inhibition of extracellular matrix (Extracellular Matrix, ECM) degradation, promotion of ECM synthesis, and inhibition of an inflammatory response, and, despite being a single ingredient, exhibits remarkably superior performance in inhibition of ECM degradation and anti-inflammatory effects compared with conventional commercial peptides (Matrixyl^{™}, SYN^{™}-AKE, etc.), thereby providing an integrated effect capable of simultaneously improving various causes of skin aging. Accordingly, the present invention may be utilized not only for preventing skin aging, improving wrinkles, and enhancing elasticity, but also for preventing or improving various inflammatory skin problems, such as improvement of acne-prone skin, alleviation of atopic dermatitis, and soothing of sensitive skin, based on the anti-inflammatory effect.

The present invention provides a method for improving a skin condition, characterized by administering or applying to the skin an effective amount of a hexapeptide having the RKWRYR sequence represented by SEQ ID NO: 1 or a hexapeptide in which the N-terminus thereof is modified with a palmitoyl group.

The present invention provides a method for preventing or treating an inflammatory skin disease, characterized by administering or applying to the skin an effective amount of a hexapeptide having the RKWRYR sequence represented by SEQ ID NO: 1 or a hexapeptide in which the N-terminus thereof is modified with a palmitoyl group.

The hexapeptide of the present invention may be prepared in formulations such as ointments, creams, lotions, and serums and may be usefully used for improvement of a skin condition or prevention and treatment of an inflammatory skin disease by directly applying the same to the skin.

### Advantageous Effects of Invention

The novel peptide according to the present invention exhibits the following effects with respect to complex causes of skin aging.

First, the peptide of SEQ ID NO: 1 of the present invention has an excellent inhibitory effect against extracellular matrix (ECM) degradation. Specifically, at a concentration of 100 ppm, the peptide inhibits collagenase activity by 70% or more and elastase activity by 80% or more, thereby effectively preventing structural collapse of the skin.

Second, the peptide of SEQ ID NO: 1 of the present invention exhibits an excellent ability to promote extracellular matrix (ECM) synthesis. Specifically, even at a concentration of 5 ppm, the peptide increases collagen synthesis by 30% or more compared with the control group, thereby contributing to improvement of skin elasticity and density.

Third, the peptide of SEQ ID NO: 1 of the present invention exhibits a strong anti-inflammatory effect. At a concentration of 5 ppm, the peptide inhibits production of nitric oxide (NO), which is an inflammation-inducing factor, by 80% or more and production of prostaglandin E2 (PGE2) by 64% or more, thereby inhibiting an inflammatory response that accelerates skin aging.

In addition, despite being a single ingredient, the peptide of SEQ ID NO: 1 of the present invention exhibits remarkably superior performance in inhibition of ECM degradation and anti-inflammatory effects compared with commercial peptides (Matrixyl^{™}, SYN^{™}-AKE, etc.), thereby providing an integrated effect capable of simultaneously improving various causes of skin aging.

Accordingly, the present invention may be utilized not only for preventing skin aging, improving wrinkles, and enhancing elasticity, but also for preventing or improving various inflammatory skin problems such as improvement of acne-prone skin, alleviation of atopic dermatitis, and soothing of sensitive skin, based on the anti-inflammatory effect.

In addition, the composition of the present invention may be applied not only to traditional cosmetic formulations such as creams, lotions, serums, and ampoules, but also to highly functional formulations such as microneedle patches, hydrogel masks, and scalp tonics, which enhance skin delivery efficiency of the active ingredient. Furthermore, the composition of the present invention has high industrial potential capable of being usefully utilized in various fields such as cosmetic compositions, pharmaceutical compositions, medicines, quasi-drugs, and medical device formulations.

### Brief Description of Drawings

FIG. 1 is a graph showing the degree of inhibition of collagenase activity according to concentrations of the peptide of SEQ ID NO: 1 of the present invention and vitamin C used as a positive control.
FIG. 2 is a bar graph showing the degree of inhibition of elastase activity according to concentrations of the peptide of SEQ ID NO: 1 of the present invention and vitamin C used as a control.
FIG. 3 is a bar graph showing the collagen biosynthesis-promoting effect when the peptide of SEQ ID NO: 1 of the present invention is treated at various concentrations.
FIG. 4 is a bar graph showing a comparison of collagen biosynthesis-promoting effects of the peptide of SEQ ID NO: 1 of the present invention, commercial peptides (Matrixyl^{™}, SYN^{™}-AKE, Argireline^{™}), and retinol.
FIG. 5 is a bar graph showing a comparison of NO production inhibitory effects of the peptide of SEQ ID NO: 1 of the present invention, commercial peptides (Matrixyl^{™}, SYN^{™}-AKE, Argireline^{™}), and retinol.
FIG. 6 is a bar graph showing a comparison of prostaglandin E2 production inhibitory effects of the peptide of SEQ ID NO: 1 of the present invention, commercial peptides (Matrixyl^{™}, SYN^{™}-AKE, Argireline^{™}), and retinol.

### Best Mode for Carrying Out the Invention

The present inventor recognized the limitations of the fragmentary approach possessed by the prior art and conducted research in order to derive an optimal amino acid sequence capable of simultaneously controlling, within one molecular structure, extracellular matrix (ECM) degradation, reduction in synthesis, and inflammatory response, which are complex causes of skin aging. In particular, based on screening technology and research know-how secured in a prior patent application (Application No. 10-2022-0092749), through subsequent research, it was confirmed that a specific amino acid sequence (SEQ ID NO: 1) simultaneously exhibits collagenase inhibitory activity, elastase inhibitory activity, collagen biosynthesis-promoting activity, and anti-inflammatory activity, thereby completing the present invention.

Hereinafter, preferred examples of the present invention will be described in detail. However, the present invention is not limited to the following examples, and various modifications and applications are possible within a range that does not depart from the technical spirit of the present invention. The following description is merely exemplary description for sufficiently understanding the contents of the present invention.

### <Example 1. Peptide Synthesis>

Using an automatic peptide synthesizer, a peptide having the amino acid sequence of the sequence number described in Table 1 below was synthesized, and the synthesized peptides were purified and isolated using C18 reverse-phase high performance liquid chromatography (HPLC) (U-3000, Thermo Fisher Scientific, USA). The peptide may be modified by binding palmitic acid to the N-terminus. The molecular structures of the synthesized peptide and the N-terminally modified palmitoyl peptide are shown in Table 1 below.

**[Table 1]**

| Sequence number | 1 |
|---|---|
| Amino acid sequence | RKWRYR |
| Molecular structure | 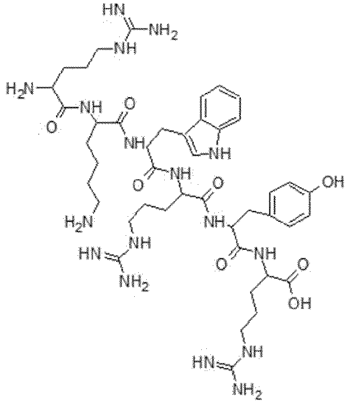 |
| N-terminal palmitoyl modification | 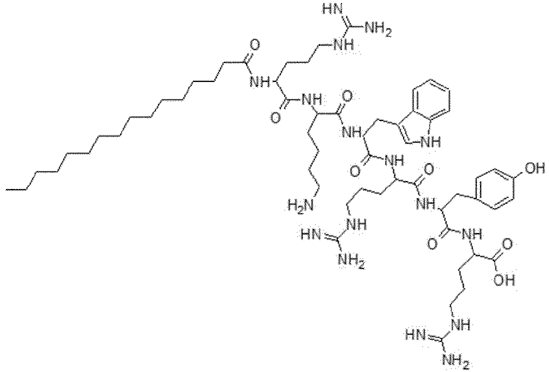 |

(Provided that R is arginine, K is lysine, W is tryptophan, and Y is tyrosine.)

### <Example 2. Activity Inhibitory Effect Against Collagenase and Elastase>

### Collagenase activity inhibition

The collagenase activity inhibitory effect according to the concentration of the peptide of SEQ ID NO: 1 of the present invention was evaluated. This experiment was performed in accordance with the method described in the manufacturer's manual (Gelatinase/Collagenase Assay Kit, Cat. No. E12055, Invitrogen^{™}, EnzChek^{™}).

The total reaction volume was 200 µl, and a black 96-well plate was used in order to minimize fluorescence interference. To each well, 110 µl of 1X reaction buffer was added, and then 20 µl of the peptide of SEQ ID NO: 1 of the present invention or vitamin C, which is a positive control substance, was added and mixed by stirring two to three times. Subsequently, 20 µl of collagenase (10 U/ml), which is an enzyme, and 50 µl of collagen type 1 (100 µg/ml), which is a substrate, were added, followed by reaction at 37°C for 1 hour. After completion of the reaction, fluorescence intensity was measured using a fluorescence microplate reader under conditions of an excitation wavelength of 495 nm and an emission wavelength of 515 nm.

The collagenase activity inhibition rate was calculated according to Mathematical Formula 1 below by comparing the fluorescence value of the group treated with the peptide of SEQ ID NO: 1 of the present invention or the positive control substance vitamin C with the fluorescence value of the untreated group in which enzyme (Collagenase) and substrate (Collagen type 1) were reacted as a reference. [Mathematical Formula 1] $Collagenase activity inhibition rate \left(%\right) = 1 - \left(\frac{fluorescence value of the test material - treated group}{fluorescence value of the untreated group}\right) \times 100$

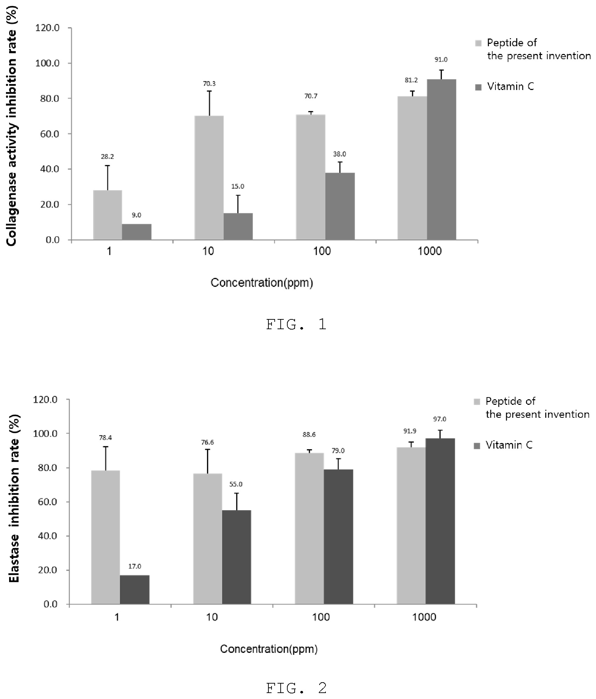

The results measured according to the above method are shown in Table 2 and FIG. 1.

**[Table 2]**

| **Test substance name** | **Concentration (ppm)** | **Collagenase activity inhibition rate (%)** |
|---|---|---|
| Peptide of SEQ ID NO: 1 | 1000 | 81.2 |
| | 100 | 70.7 |
| | 10 | 70.3 |
| | 1 | 28.2 |
| Vitamin C | 1000 | 91 |
| | 100 | 38 |
| | 10 | 15 |
| | 1 | 9 |

As shown in Table 2 and FIG. 1, at concentrations of 100 ppm or less, the collagenase activity inhibitory effect of the peptide of SEQ ID NO: 1 according to the present invention was superior to that of vitamin C, and at a concentration of 10 ppm, vitamin C recorded a collagenase inhibition rate of 15%, whereas the peptide of SEQ ID NO: 1 of the present invention at the same concentration exhibited a collagenase inhibition rate of 70.3%. In the same manner as above, collagenase activity was measured using commercial peptides SYN^{™}-AKE, Matrixyl^{™}, Argireline^{™}, and retinol as control groups, and the results are shown in Table 3.

**[Table 3]**

| **Test substance name** | **Concentration (ppm)** | **Collagenase activity inhibition rate (%)** |
|---|---|---|
| Peptide of SEQ ID NO: 1 | 100 | 74.2 |
| | 10 | 75.6 |
| | 1 | 23.2 |
| SYN^{™}-AKE | 100 | 16 |
| | 10 | 4.4 |
| | 1 | 6 |
| Matrixyl^{™} | 100 | 6.1 |
| | 10 | - |
| | 1 | - |
| Argireline^{™} | 100 | - |
| | 10 | - |
| | 1 | - |
| Retinol | 100 | - |
| | 10 | - |
| | 1 | - |

As shown in Table 3, the peptide of SEQ ID NO: 1 according to the present invention exhibited a remarkable collagenase inhibitory effect compared with commercial peptides SYN^{™}-AKE, Matrixyl^{™}, Argireline^{™}, and retinol, and particularly exhibited a collagenase activity inhibitory effect that was 17 times that of SYN^{™}-AKE at a concentration of 10 ppm, 4.6 times that of SYN^{™}-AKE at a concentration of 100 ppm, and 12.2 times that of Matrixyl^{™}.

### Elastase activity inhibition

The elastase inhibitory activity according to the concentration of the peptide of SEQ ID NO: 1 of the present invention was confirmed. Elastase activity was measured according to the manufacturer's manual (Elastase Assay Kit, Cat. No. E12056, Invitrogen^{™}, EnzChek^{™}) by a method similar to the above example.

The total reaction volume was 200 µl, and a black 96-well plate was used in order to minimize fluorescence interference. To each well, 110 µl of 1X reaction buffer was added, and then 20 µl of the peptide of SEQ ID NO: 1 of the present invention or vitamin C, which is a positive control substance, was added and mixed by stirring two to three times. Thereafter, 100 µl of enzyme (0.1 U/ml) and 50 µl of DQ elastin (100 µg/ml), which is a substrate, were added, followed by reaction at 37°C for 2 hours. After completion of the reaction, fluorescence intensity was measured using a fluorescence microplate reader under conditions of an excitation wavelength of 495 nm and an emission wavelength of 515 nm. The elastase activity inhibition rate was calculated as shown in the following [Mathematical Formula 2], and the results are shown in Table 4 and FIG. 2.

[Mathematical Formula 2] $Elastase activity inhibition rate \left(%\right) = 1 - \left(\frac{fluorescence value of the test material - treated group}{fluorescence value of the untreated group}\right) \times 100$

**[Table 4]**

| **Test substance name** | **Concentration (ppm)** | **Elastase activity inhibition rate (%)** |
|---|---|---|
| Peptide of SEQ ID NO: 1 | 1000 | 91.9 |
| | 100 | 88.6 |
| | 10 | 76.6 |
| | 1 | 78.4 |
| Vitamin C | 1000 | 97 |
| | 100 | 79 |
| | 10 | 55 |
| | 1 | 17 |

As shown in Table 4 and FIG. 2, at concentrations of 100 ppm or less, the elastase activity inhibitory effect of the peptide of SEQ ID NO: 1 according to the present invention was superior to that of vitamin C, and particularly at a concentration of 1 ppm, vitamin C recorded an elastase inhibition rate of 17%, whereas the peptide of SEQ ID NO: 1 of the present invention at the same concentration exhibited elastase inhibition of 78.4%. In the same manner as above, elastase activity was measured using commercial peptides SYN^{™}-AKE, Matrixyl^{™}, Argireline^{™}, and retinol as control groups, and the results are shown in Table 5.

**[Table 5]**

| **Test substance name** | **Concentration (ppm)** | **Elastase activity inhibition rate (%)** |
|---|---|---|
| Peptide of SEQ ID NO: 1 | 100 | 88.7 |
| | 10 | 85.8 |
| | 1 | 66.6 |
| SYN^{™}-AKE | 100 | - |
| | 10 | - |
| | 1 | - |
| Matrixyl^{™} | 100 | - |
| | 10 | - |
| | 1 | - |
| Argireline^{™} | 100 | - |
| | 10 | 3.2 |
| | 1 | - |
| Retinol | 100 | - |
| | 10 | - |
| | 1 | - |

As shown in Table 5, the peptide of SEQ ID NO: 1 according to the present invention exhibited a remarkable elastase inhibitory effect compared with the commercial peptide Argireline^{™}, and particularly exhibited an elastase inhibitory effect that was 26.8 times that of Argireline^{™} at a concentration of 10 ppm.

### <Example 3. Collagen Biosynthesis-Promoting Effect>

### Promotion of collagen biosynthesis

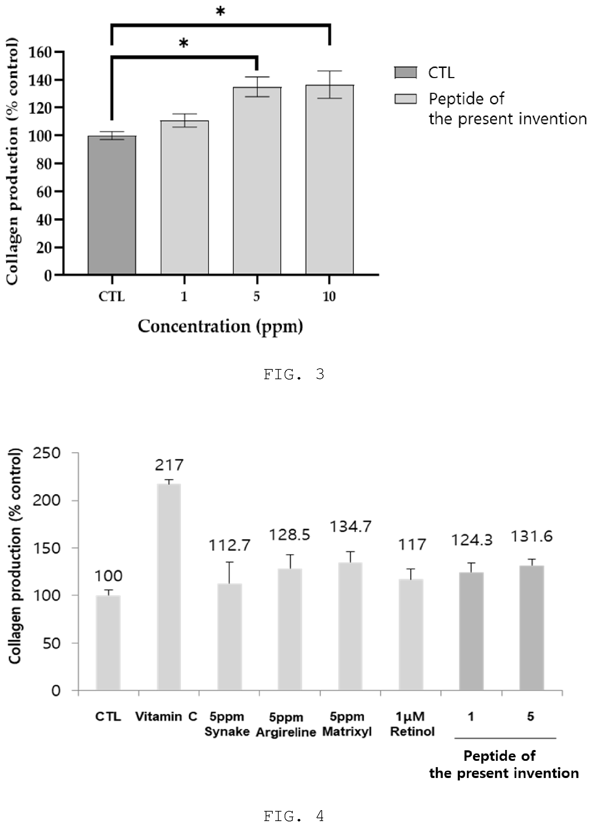

It was confirmed whether the peptide of SEQ ID NO: 1 according to the present invention promotes collagen biosynthesis. The main procedures of the intracellular collagen biosynthesis test were performed according to the guidelines of the Korea Food and Drug Administration. Human dermal fibroblasts (human Dermal fibroblast, HDF) derived from human skin were seeded in a 48-well plate at a concentration of 2 to 5 × 10^4 cells/ml and cultured for 24 hours. The growth medium was removed, an FBS-free medium containing the peptide of SEQ ID NO: 1 was added, and after culturing for 24 hours, the cell culture medium was collected and subjected to an ELISA assay. The human procollagen 1α1 DuoSet ELISA kit used in this test was a product of R&D Systems, and evaluation was performed according to the experimental protocol provided by the manufacturer. The results are shown in Table 6 and FIG. 3.

**[Table 6]**

| **Test substance name** | **Concentration (ppm)** | **Collagen biosynthesis (%)** |
|---|---|---|
| Peptide of SEQ ID NO: 1 | 0 (control group, CTL) | 100 |
| | 1 | 110.91 |
| | 5 | 134.94 |
| | 10 | 136.56 |

As shown in Table 6 and FIG. 3, compared with the control group, the amount of collagen I production was statistically significantly increased by 10.9% at 1 ppm, 34.9% at 5 ppm, and 36.5% at 10 ppm. Accordingly, it could be confirmed that the peptide of SEQ ID NO: 1 has an effect of increasing collagen I synthesis.

In the same manner as above, the effects on collagen production were confirmed using the peptide of SEQ ID NO: 1 according to the present invention and commercial peptides SYN^{™}-AKE, Matrixyl^{™}, Argireline^{™}, retinol, and vitamin C as control groups, and the results are shown in FIG. 4.

As shown in FIG. 4, it was confirmed that the peptide of SEQ ID NO: 1 according to the present invention increases collagen biosynthesis to the same or higher level than commercial peptides, and particularly induced a remarkable increase in collagen synthesis compared with the control group, at 124.3% and 131.6% at 1 and 5 ppm, respectively.

### <Example 4. Anti-inflammatory Effect>

In order to confirm the anti-inflammatory efficacy of the peptide of SEQ ID NO: 1 according to the present invention, the ability to inhibit production of inflammatory mediators nitric oxide (Nitric Oxide) and prostaglandin E2 (Prostaglandin E2) was evaluated in RAW 264.7 cells, a mouse-derived macrophage cell line in which an inflammatory response was induced with lipopolysaccharide (Lipopolysaccharide, LPS).

### Nitric oxide (NO) production inhibitory effect

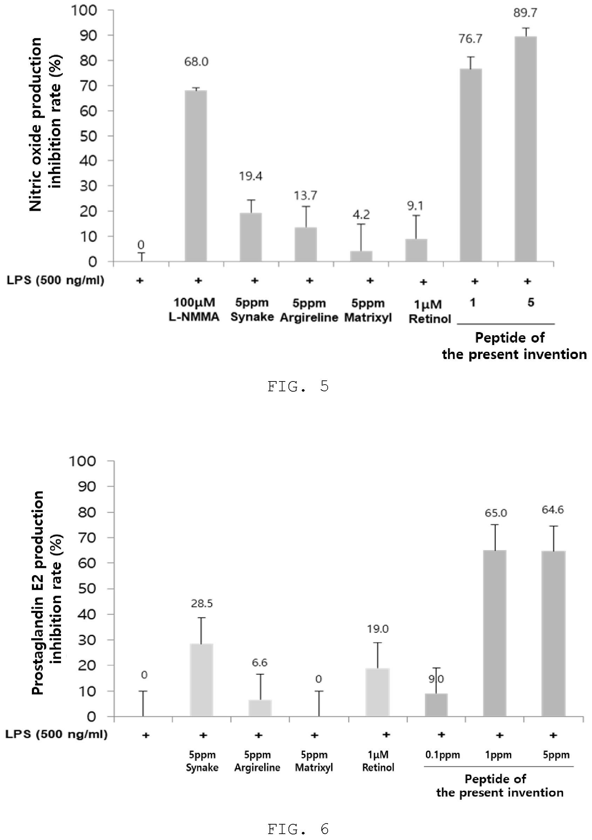

Raw 264.7 cells, which are mouse macrophage cells, were added to a 24-well tissue culture plate at a concentration of 1 to 2 × 10⁵ cells/ml using DMEM medium containing 10% fetal bovine serum and 1% penicillin/streptomycin, and cultured for one day in a 5% CO₂ incubator. The experiment was performed by the GRIESS method. The medium was removed, starvation was induced with serum-free medium for 12 hours, and then L-NMMA 100 µM, SYN^{™}-AKE 5 ppm, Matrixyl^{™} 5 ppm, Argireline^{™} 5 ppm, retinol 1 µM, and the peptide of SEQ ID NO: 1 of the present invention at 1 and 5 ppm were pretreated for 30 minutes, followed by addition of lipopolysaccharide at a concentration of 500 ng/ml and culturing for 18 hours. After culturing, the supernatant was collected and transferred to a 96-well plate, GRIESS reagent (Sigma, G4410) was added, reaction was performed at room temperature for 15 minutes, and absorbance at 540 nm was measured using an ELISA reader. The results were converted into a NO production inhibition rate relative to the untreated group and are shown in FIG. 5.

As shown in FIG. 5, the peptide of SEQ ID NO: 1 according to the present invention inhibited NO production by 76.7% and 89.7%, respectively, upon treatment at concentrations of 1 and 5 ppm, and exhibited a NO production inhibition rate superior to that of commercial peptides, thereby confirming a remarkable anti-inflammatory effect.

### Prostaglandin E2 (PGE2) production inhibitory effect

RAW 264.7 cells cultured under the same conditions as in the above example were pretreated for 30 minutes with SYN^{™}-AKE 5 ppm, Matrixyl^{™} 5 ppm, Argireline^{™} 5 ppm, retinol 1 µM, and the peptide of SEQ ID NO: 1 of the present invention at 0.1, 1, and 5 ppm, followed by treatment with LPS (500 ng/ml) and additional culturing for 18 hours. The culture supernatant was collected and subjected to an ELISA assay. The prostaglandin E2 Biotrak^{™} EIA kit used in this test was a product of GE Healthcare Life Sciences (Seoul, Korea), and evaluation was performed according to the experimental protocol provided by the manufacturer. The results were converted into a PGE2 production inhibition rate relative to the untreated group and are shown in FIG. 6.

As shown in FIG. 6, the peptide of SEQ ID NO: 1 according to the present invention inhibited PGE2 production by 65% and 64.6%, respectively, upon treatment at concentrations of 1 and 5 ppm, and exhibited a PGE2 production inhibition rate superior to that of commercial peptides SYN^{™}-AKE, Matrixyl^{™}, and Argireline^{™}, thereby confirming a remarkable anti-inflammatory effect.

### Sequence Listing

Sequence listing electronic file attached

## Claims

1. A hexapeptide having the RKWRYR sequence represented by SEQ ID NO: 1.
(Provided that R is arginine, K is lysine, W is tryptophan, and Y is tyrosine.)

2. The hexapeptide according to claim 1,
wherein the hexapeptide is a hexapeptide in which the N-terminus is modified with a palmitoyl group.

3. The hexapeptide according to claim 1 or claim 2,
wherein the hexapeptide simultaneously exhibits collagenase-inhibitory activity, elastase-inhibitory activity, collagen synthesis-promoting activity in fibroblasts, nitric oxide (NO) production-inhibitory activity, and prostaglandin E2 (PGE2) production-inhibitory activity.

4. A pharmaceutical composition for preventing or treating an inflammatory skin disease, comprising the hexapeptide according to claim 1 or claim 2.

5. The pharmaceutical composition according to claim 4,
wherein the hexapeptide inhibits production of nitric oxide (Nitric Oxide, NO) or prostaglandin E2 (Prostaglandin E2, PGE2).

6. The pharmaceutical composition according to claim 4,
wherein the inflammatory skin disease is at least one selected from acne, atopic dermatitis, contact dermatitis, allergic dermatitis, seborrheic dermatitis, psoriasis, photosensitive dermatitis, and rosacea.

7. A cosmetic composition for improving a skin condition, comprising the hexapeptide according to claim 1 or claim 2.

8. The cosmetic composition according to claim 7,
wherein the improvement of the skin condition is improvement of skin elasticity, improvement of skin wrinkles, prevention of skin aging, enhancement of skin elasticity, or improvement of inflammatory skin.

9. The cosmetic composition according to claim 8,
wherein the improvement of inflammatory skin is improvement of acne, atopic dermatitis, contact dermatitis, allergic dermatitis, seborrheic dermatitis, psoriasis, photosensitive dermatitis, or rosacea.

10. The cosmetic composition according to claim 7,
wherein the improvement of the skin condition is caused by at least one mechanism selected from the group consisting of inhibition of extracellular matrix (Extracellular Matrix, ECM) degradation, promotion of ECM synthesis, and inhibition of an inflammatory response.

11. The cosmetic composition according to claim 7,
wherein the improvement of the skin condition is caused by inhibition of collagenase activity, inhibition of elastase activity, promotion of collagen synthesis in fibroblasts, inhibition of production of nitric oxide (Nitric Oxide, NO), or inhibition of production of prostaglandin E2 (Prostaglandin E2, PGE2).

12. A method for improving a skin condition, **characterized by** administering or applying to the skin an effective amount of a hexapeptide having the RKWRYR sequence represented by SEQ ID NO: 1 or a hexapeptide in which the N-terminus thereof is modified with a palmitoyl group.

13. A method for preventing or treating an inflammatory skin disease, **characterized by** administering or applying to the skin an effective amount of a hexapeptide having the RKWRYR sequence represented by SEQ ID NO: 1 or a hexapeptide in which the N-terminus thereof is modified with a palmitoyl group.
